# EUROPEAN PATENT SPECIFICATION

(11) **EP 2 040 649 B1**
(45) Date of publication and mention of the grant of the patent: **24.10.2012**
(21) Application number: 07810140.9
(22) Date of filing: 02.07.2007
(51) Int. Cl.: A61F 2/66

(54) **PROSTHETIC FOOT**
FUSSPROTHESE
PROTHÈSE DE PIED

(30) Priority: 03.07.2006 US 817700 P; 30.11.2006 US 861716 P; 29.06.2007 US 819844
(43) Date of publication of application: 01.04.2009
(73) Proprietor: Össur hf, 110 Reykjavik (IS)
(72) Inventor: ASGEIRSSON, Sigurdur, 210 Gardabaer (IS); OLAFSSON, Gudlaugur, 900 Vestmanneaeyjar (IS); INGIMARSSON, Gudni, 112 Reykjavik (IS)
(74) Representative: Höhfeld, Jochen
(86) International application number: PCT/US2007/015338
(87) International publication number: WO 2008/005424

(56) References cited:
- EP-A1- 0 487 852
- WO-A2-2005/117749
- US-A- 2 556 525
- US-A- 4 652 266
- US-A- 4 865 612
- US-A- 5 007 938
- US-A- 5 888 239
- US-B1- 6 261 324

## Description

**FIELD OF THE INVENTION**

The present invention relates generally to the field of prosthetic devices, and more particularly to prosthetic feet and footplates for use in therein.

**BACKGROUND**

In the field of prosthetics, many significant advances in construction and design of prosthetic limbs have been made possible due to improved materials and manufacturing capability. In particular, prosthetic feet and footplates for use therein have undergone large improvements in both design and construction.

The use of lightweight plastics and composite materials in prosthetic feet and footplates represents a significant improvement over the previous designs, which typically included solid blocks of wood that were cosmetically shaped. In addition, the study of biomechanics and the gait cycle have allowed for improved designs that more closely simulate the mechanics and responses of the human foot.

While the current designs of prosthetic feet and footplates represent an advance over the previous designs, a majority of the current designs have become quite complex. Due to the complexity of the designs, and material and manufacturing costs, current prosthetic feet are relatively expensive.

One exemplary prosthetic foot is disclosed in U.S. patent no. 2,556,525, in which a foot portion is composed of an outer layer of soft material, such as sponge rubber, and a core of relatively stiff material, such as vulcanized rubber. A spring steel anchor plate is embedded within the core.

Another example of a prosthetic foot is disclosed in U.S. patent no. 4,865,612, in which an artificial foot includes a hollow, rigid, compression-molded composite keel made from aramid fiber-reinforced plastic for connection to a prothesis and to a composite carbon-fiber material acting as a spring for improving the energy absorbing and releasing characteristics of the foot to aid movement of the amputee. The spring includes a pair of thin, forwardly-extending composite spring members capable of lateral movement with respect to each other and connected to the keel. An auxiliary deflection spring or plate is interposed between the primary deflection plate and the keel and includes a forwardly-extending upwardly-oriented portion to aid in energy absorption and release during flexing of the foot. The plates are surrounded by a suitable abrasion resistant material, such as a Kevlar sock, surrounded by a low density flexible foam urethane. A medium density flexible foam urethane wedge is interposed beneath the heel portion of the keel. The composite construction is surrounded by an outer shell formed in the shape cosmetically to resemble a foot.

Yet another example of a prosthetic foot is disclosed in U.S. patent no. 6,261,324, where a foot prosthesis incorporates a single composite member having a forefoot portion beneath which is molded a unitary elastomeric sole that projects rearwardly from the composite member to define a heel. The elastomeric sole also projects laterally from the composite member so as to provide a degree of lateral stability that matches the inversion/eversion resistance of the normal human foot. In addition, a separate height adjuster and/or stiffness adjuster can be removably attached to the underside of the elastomeric sole, to enable the user to selectively adjust the prosthesis' heel height and/or stiffness.

In another example of a prosthetic foot, disclosed in U.S. patent no. 5,007,938, where, within the plantar area thereof, the artificial foot for a leg prosthesis is provided with a plate-shaped reinforcing member embedded in the molded foot part. The member includes two superimposed upper and lower leaf springs possessing different lengths, between which a carbon fiber-reinforced plastic leaf spring is disposed extending. The latter extends into the ball of the foot area and is offset at the front end. Sliding inserts of polyethylene and each face of the plastic leaf spring and between the upper and lower leaf springs produce a constant metatarsal elasticity even during continuous stress.

In still another example of a prosthetic foot disclosed in EP 0 487 852 A1, the prosthetic foot includes a prosthesis body having a toe region disposed in a front part of the prosthetic foot, and a one-piece insert which is provided in the prosthesis body and which absorbs and transmits stresses imposed on the prosthesis body. The insert enables at least a plantar and dorsal deflection as well as axial compression of the prosthesis body, and has an approximately S-shaped design in the longitudinal section of the prosthetic foot. The insert includes a top horizontal section which forms a top edge of the prosthetic foot, and a bottom horizontal section which is of an extended construction compared with the top section and which has a free end which extends into the toe region of the prosthesis body, so that a high elasticity and a high energy storage capacity of the front part of the prosthetic foot are achieved. A front inclined section extends at an obtuse angle from the top section to form a rigid angular element. A central section functions as a leaf spring and has a front end which is connected to a lower end of the angular element and has a rear end. An approximately semi-circular rear connecting section is connected to the bottom section and to the rear end of the central section.

In another exampled disclosed in U.S. patent no. 4,652,266, a prosthetic foot includes an articulated heel. The heel comprises a plurality of links and has energy-storing resilient devices at adjacent ends of the links.

In yet another example of a prosthetic foot disclosed in U.S. patent no. 5,888,239, a foot prosthesis has an inner spring that extends from the heel area to the front area of the foot. The foot spring is enclosed within a cosmetic lining and is joined by a threaded bolt to an adapter with a clamping connection for removably joining the foot prosthesis to a prosthesis tube. The adapter is supported by its lower face, which is designed as a cylinder segment and is provided with serrations, on a joining surface of the inner spring. The joining surface of the inner spring has a shape corresponding to the lower face of the adapter, and has serrations for engaging the adapter's serrations. The upper end of the adapter is designed as a peg which is inserted into the prosthesis tube. The threaded bolt is inserted through a slot that extends in the longitudinal direction of the foot and has a width corresponding to the diameter of the bolt. The lower end of the threaded bolt is supported on the lower side of the inner spring, the lower side being designed as a cylinder segment in the area of the slot. The upper end of the threaded bolt that projects above the adapter peg acts upon the clamping connection such that the spreading element of the clamping connection, which is arranged in a ring-shaped gap between the adapter peg and the prosthesis tube, is actuated when the threaded bolt is tightened.

In still another example of a prosthetic foot disclosed in WO 2005/117749, the foot prosthesis includes various structural features that provide the foot with advantageous rollover properties. In certain embodiments, the foot guides rollover toward the medial side. For example, an asymmetrical upper element and a correspondingly shaped resilient ankle member support more of the wearer's weight on the lateral side as the foot rolls over. In another embodiment, stiffeners added to the resilient ankle member increase the stiffness on the lateral side relative to the medial side. In certain other embodiments, the foot provides progressively increasing support from mid stance through toe off. For example, a gap between the resilient ankle member and the lower element closes during the later portion of the wearer's gait. The closing gap increases a contact area between the resilient ankle member and the lower element, providing progressively increasing support. In another embodiment, the foot includes a gap between a lower front edge of an attachment adapter and the upper element. The gap may be filled with a resilient material.

A prosthetic foot according to the preamble appended claim 1 is known in the prior art, for example from U.S. patent no. 2,556,525.

Due to the relatively high cost of many current prosthetic feet, people in developing and underdeveloped countries and regions of the world have limited or no access to prosthetic feet and footplates. This is especially unfortunate, as many underdeveloped and developing regions are recovering from years of civil wars and regional conflicts where the use of landmines has been rampant. Since landmines have a tendency to cause injuries to the lower extremities, thee is a large need for prosthetic feet and footplates in regions that are affected by this danger. However, most of the people in developing and underdeveloped regions who are in need of prosthetic limbs are precluded access to most of the current designs because of their economic situation.

Additionally, users of prosthetic feet all over the world may not need all of the additional performance characteristics provided by complex and expensive prosthetic feet. Many users of prosthetic feet simply require a prosthetic foot that provides comfort and stability, as opposed to a prosthetic foot that must closely simulate all of the mechanics of a human foot. For example, low activity users do not require a prosthetic foot that will allow them to run and jog. An example of a low activity user may be an elderly user who may not need a complex prosthetic foot that closely simulates the behavior of the human foot, but instead may only require a prosthetic foot that provides some simulation of the human foot while providing sufficient comfort and stability.

Thus, it would be advantageous to provide functional and light-weight prosthetic feet that may be manufactured economically while providing all of the basic necessary attributes required of a prosthetic foot. Such prosthetic feet would be more accessible to more people of the world than most current designs.

**SUMMARY**

In order to provide low cost and improved prosthetic feet, a prosthetic foot as defined in claim 1 is provided. Preferred embodiments are defined in the dependent claims.

One embodiment of a prosthetic foot includes a resilient footplate embedded within a first foam element that has a specific density. The footplate is defined by proximal and distal surfaces, as well as anterior and posterior portions, with a terminal end located in the posterior portion. A second foam element is bonded to the distal surface of the posterior portion of the footplate and is also embedded within the first foam element. The second foam element has a density that is higher than the density of the first foam element. The second foam element also has a recess in the proximal surface of the element. Due to the recess in the second foam element, an accommodation space is formed between the proximal surface of the second foam element and the distal surface of the footplate. The first foam element fills in the accomodation space.

In another embodiment, the prosthetic foot may have a tough outer shell that is scuff, puncture and tear resistant, and which defines a cosmesis that encloses the first and second foam elements.

In yet another embodiment, the prosthetic foot may incorporate a pyramid that is retained by a pyramid adapter, wherein at least one attachment bolt secures the pyramid and the pyramid adapter to the resilient footplate. In this embodiment, each attachment bolt includes a bolt head configured to engage the distal surface of the footplate, such that each bolt head is accommodated within the space defined between the second foam element and the footplate. At least a portion of the pyramid adapter may be embedded within the first foam element. Of course, any suitable mechanism for connecting the prosthetic foot to a prosthetic limb, socket, or pylon may be utilized in place of the pyramid connector described.

According to the invention a clearance space is located between the proximal surface of the second foam element and the distal surface of the footplate, such that the first foam element fills in the recess and encases each bolt head.

In another variation, the second foam element may have an extending portion that extends posterior to the terminal end of the resilient footplate.

In another embodiment, which is not part of the invention, the prosthetic foot includes a resilient footplate embedded within a first foam element having a first density. The prosthetic foot also includes a resilient footplate embedded within a second foam element having a second density that may be the same density as the first foam element. The second density may also be greater than the first density. Again, the footplate may be defined by proximal and distal surfaces, as well as anterior and posterior portions.

The first foam element may be disposed along the proximal surface of the footplate and the second foam element may be provided along the distal surface of the footplate. A tough outer shell that is scuff, puncture and tear resistant defines a cosmesis that encloses the first and second foam elements. A third foam element having a third density may be provided such that it extends through a distal posterior surface of the cosmesis and into a distal posterior portion of the second foam element. The third density of the third foam element may be greater than the densities of the first foam element and the second foam element. The third foam element has proximal and distal surfaces and may be trapezoidal in shape, where the distal surface is larger than the proximal surface.

Again, a pyramid adapter or other suitable adapter structure may be provided, and the second foam element may have a recess in the proximal surface thereof such that a space for accommodating the connection components is formed between the second foam element and the footplate.

In alternative constructions of footplates used in the embodiments discussed above, a combination of materials may be used, such as layers of polymers and carbon fiber composites.

The numerous advantages, features and functions of the various prosthetic feet will become readily apparent and better understood in view of the following description, appended claims, and accompanying drawings. The following description is not intended to limit the scope of the prosthetic feet, but instead merely provides exemplary embodiments for ease of understanding.

**BRIEF DESCRIPTION OF THE DRAWINGS**

Fig. 1 is a cross-sectional view of an embodiment of a prosthetic foot.

Fig. 2 is a cross-sectional view of another embodiment of a prosthetic foot.

Fig. 3 is a cross-sectional view of still another embodiment of a prosthetic foot.

Fig. 4 is a cross-sectional view of yet another embodiment of a prosthetic foot, which is not part of the invention.

In the various figures, similar elements are provided with similar reference numbers. It should be noted that the drawing figures are not necessarily drawn to scale, but instead are drawn to provide a better understanding of the components thereof.

**DETAILED DESCRIPTION OF VARIOUS EMBODIMENTS**

A. Environment and Context of the Various Embodiments

The prosthetic feet in accordance with this disclosure are designed for implementation in connection with typical artificial limb hardware including prosthetic sockets, prosthetic knees, pylons and the like.

The prosthetic feet are mountable to a distal end of a pylon using a typical pyramid connection used for such applications with an adapter allowing adjustment of foot-to-pylon angles in accordance with typical adjustment features found on prosthetic feet currently offered for sale. Alternatively, clamping or threaded connections, or any other suitable connections may be used in place of the pyramid connection. Exemplary connection mechanisms are disclosed in, for example, U.S. patent no. 6,811,571, granted November 2, 2004, and U.S. publication no. 2005/0234563, published October 20, 2005.

Features of the prosthetic feet include a central load bearing resilient footplate to which a connection mechanism, such as a pyramid and pyramid adapter, is secured by an attachment bolt or bolts. The footplate typically is embedded in a composite foam matrix comprising dual density, or generally a plurality of density foam elements, that provide an interface between the footplate and the surface on which a patient is supported.

1. Gait Cycle

In order to better understand the operation and benefits of the prosthetic feet described herein, a basic discussion of the gait cycle is required. The gait cycle defines the movement of the leg between successive heel contacts of the same foot. The gait cycle has two phases: stance and swing. Of particular interest in the field of prosthetic feet is the stance phase, which includes five time periods: heel-strike or initial contact, loading response, mid-stance, terminal stance, and pre-swing or toe-off.

It is during the stance phase that the mechanics of a prosthetic foot come into play. Any suitable prosthetic foot must be able to provide some cushioning during heel-strike, and some energy storage at least during mid-stance, terminal stance, and toe-off. In addition, a prosthetic foot must provide stability during mid-stance and terminal stance, at which time the entire weight of a user is transmitted through the prosthetic foot to a supporting surface.

Conventional prosthetic feet perform all of these functions, but with the tradeoff of expensive and complex designs. The embodiments of the prosthetic feet of this disclosure provide all of the basic attributes required of a prosthetic foot in an economical, light-weight design that may be economically manufactured.

2. Definitions

For further ease of understanding the prosthetic feet as disclosed herein, a description of a few terms is necessary. As used herein, the term "proximal" has its ordinary meaning and refers to a location that is closer to the heart than another location. Likewise, the term "distal" has its ordinary meaning and refers to a location that is further from the heart than another location. The term "posterior" also has its ordinary meaning and refers to a location that is behind or to the rear of another location. Lastly, the term "anterior" has its ordinary meaning and refers to a location that is ahead of or to the front of another location.

B. Detailed Description of a First Embodiment

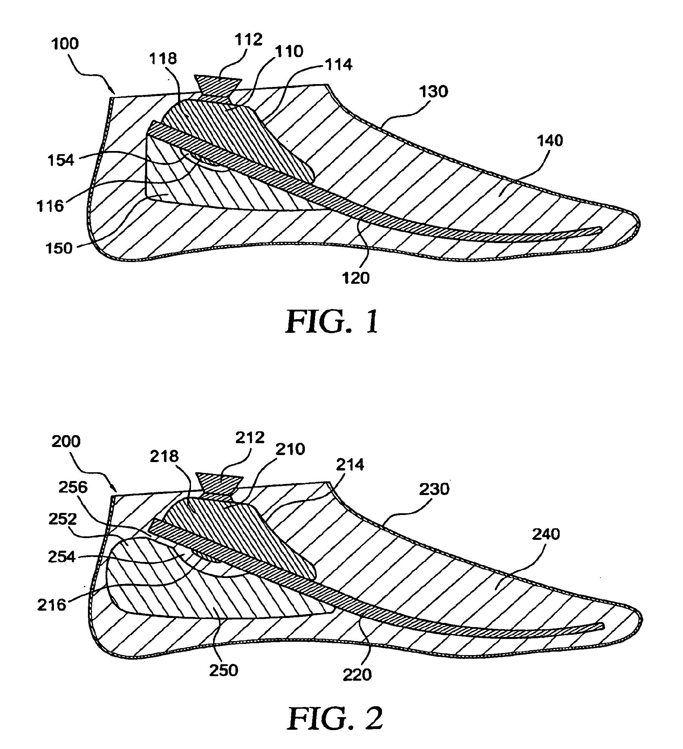

A first embodiment of a prosthetic foot **100** is shown in Fig. 1. The prosthetic foot **100** is constructed around a resilient footplate 120. The footplate **120** is appropriately shaped and configured to provide load bearing support and prosthetic foot characteristics permitting smooth ambulation.

Thus, the footplate **120** may be substantially planar, or may include one or more slight or gradual curves. The footplate **120** may include at least one recessed portion or cut out (not shown) in the anterior portion of the footplate **120** in order to provide what is known in the art as a "sandal toe," which allows a user to wear conventional toe strap sandals on the prosthetic foot, in addition to traditional footwear that covers the entire foot.

In an exemplary configuration, the footplate **120** may be inclined at an angle of twenty degrees with respect to the supporting surface or ground. Of course, any suitable orientation that provides the desired responsive characteristics may be utilized.

The footplate **120** is resilient so that some flexure may occur, but upon removal of loading, the footplate **120** returns to an unloaded and unflexed state. In order to accomplish this, the footplate **120** may be manufactured from carbon, carbon fiber composites, plastics, fiber reinforced plastics, molded chopped fibers, laminates, or any other suitable material.

Exemplary materials and constructions for the footplate **120** are described in U.S. patent no. 6,280,479, granted August 28, 2001, U.S. patent no. 5,993,488, granted November 30, 1999, and U.S. patent no. 5,800,569, granted September 1, 1998.

In addition, a footplate **120** may be provided having a variety of the aforementioned materials in combination. For example, the footplate **120** may include layers of polymers and carbon fiber composites.

An exemplary attachment mechanism **110** is provided on a proximal surface of the footplate **120** and comprises a pyramid connection **112** and a pyramid adapter **114.** The pyramid connection **112** is configured to be connected to a prosthetic limb support structure such as a pylon (not shown). The pyramid connection **112** and the pyramid adapter **114** may be of the conventional pyramid connections known in the art. Alternatively, the connection mechanism **110** may be of the tube and clamp type, may be a threaded connection, or comprise any other known attachment mechanisms.

Exemplary commercial embodiments of some connection types are available as part numbers A-135100, A-235300, A-335100, and A-435120 all available from Össur hf., Reykjavik, Iceland.

The connection mechanism **110** may include an intermediate portion **118** for engaging the proximal surface of the footplate **120,** and receiving at least one attachment bolt **116** for adjustably and firmly retaining the connection mechanism **110** in engagement with the footplate **120.** The intermediate portion **118** may have suitable coefficient of friction characteristics so that the pyramid adapter **114** will frictionally engage the footplate **120** in a manner so that the connection mechanism **110** has little to no rotational movement with respect to the footplate **120.** The proximal surface of the footplate **120** may also be provided with a suitable coefficient of friction so as to prevent slippage between the footplate **120** and the connection mechanism **110.** Of course, the use of multiple attachment bolts **116** further limits the relative movement between the footplate **120** and the connection mechanism **110,** but adds weight to the prosthetic foot.

Preferably, a first foam element **140** surrounds and embeds the footplate **120.** The first foam element **140** may have a density such that the first foam element **140** stably collapses under normal loading conditions, and thus defines a stiffness for the first foam element **140.** In an exemplary embodiment the stiffness, as defined by the hardness of the first foam element **140,** may be in the range of 45-55 on the Shore A scale. The foam matrix may be injection molded directly around the footplate **120,** or may be molded separately and bonded to the footplate **120.** The foam matrix may be any suitable open or closed cell polymer foam, such as open cell polyurethane foam.

A second foam element **150** is further embedded within the first foam element **140.** The second foam element **150** may be bonded to the distal surface of the footplate **120** prior to being embedded within the first foam element **140.** The second foam element **150** includes a proximal surface that has the same width as the footplate **120** in order to provide stability and may be adhesively bonded to the distal surface of the footplate **120** in a known manner. Any suitable adhesive or bonding technique may be used.

As can be seen from Fig. 1, the second foam element **150** is only bonded to a posterior portion of the footplate **120,** and not across the entire distal surface of the footplate **120.** In alternative embodiments, the second foam element **150** may be bonded across the entire proximal surface of the second foam element **150,** or across only a portion of the proximal surface thereof.

The second foam element **150** has a higher density than the density of the first foam element **140,** and thus has a higher stiffness. In other words, the second foam element **150** will stably collapse under a higher loading than the first foam element **140.** For example, the second foam element **150** may have a stiffness, as defined by the hardness of the second foam element **150** of about 60 on the Shore A scale. Of course, any suitable second foam element **150** having a higher stiffness than the first foam element **140** may be used.

The second foam element may be any suitable open or closed cell polymer foam, such as open cell polyurethane foam, and may be made from the same or different foam from the first foam element **140.**

The second foam element **150** includes a recessed portion **154** in the proximal surface thereof. The recess **154** may span the entire proximal surface from both sides of the second foam element **150,** or may be wholly contained between the sides of the second foam element **150.** When the second foam element **150** is placed in contact with the distal surface of the footplate **120,** the recessed portion forms an accommodation space between the footplate **120** and the second foam element **150.**

The accommodation space is configured to accommodate the head of each attachment bolt **116.** Thus, bonding the second foam element **150** to the distal surface of the footplate **120** is made easier, since the proximal surface of the second foam element **150** will correspondingly mate flushly with the distal surface of the footplate **120** in both the posterior and anterior portions. Therefore, the attachment bolt head does not cause an imbalance mating between the second foam element **150** and the footplate **120.** In an alternative configuration, a discrete recess may be provided for each attachment bolt head.

As can be seen in Fig. 1, the second foam element **150,** as well as the footplate **120,** and at least a portion of the pyramid connector **114** are all embedded within the foam matrix that defines the first foam element **140.** A tough outer shell **130** that is scuff, puncture, and tear resistant may be provided around and encasing the first foam element **140** in order to provide a cosmetically pleasing appearance for the prosthetic foot **100.** Such coverings are typically called a "cosmesis," and may be dyed or manufactured in different colors to represent different skin tones, as is known in the art.

The cosmesis shell **130** may be a separate outer covering that encases the first foam element **140,** and thus the remaining components of the prosthetic foot **100.** Alternatively, the outer surface of the first foam element **140** may itself be treated, for example under applied heat, in order to form the cosmesis shell **130** integrally with the first foam element **140.** In the instance where the cosmesis shell **130** is formed integral with the first foam element, the stiffness of the cosmesis shell **130** may be in the range of 45-55 on the Shore A scale.

The second foam element **150** has a suitable higher density than the density of the first foam element **140** due to the fact that it must absorb heel-strike stresses and provide appropriate spring-action and cushioning that cooperates with the characteristics of the resilient footplate **120** within the prosthetic foot **100.** The resilient footplate **120** provides the major support and toe-off spring action required for a prosthetic foot while the combination of the first foam element **140** and the second foam element **150** provide the heel-strike characteristics desired of a prosthetic foot. It will be noted that the resilient footplate **120** in this embodiment provides limited heel energy absorption or energy return and it is intended that the second foam element **150** in combination with the surrounding first foam element **140** will provide such desired heel-strike shock absorption and energy return functions.

Providing a prosthetic foot **100** according to this construction yields numerous advantages. One advantage is that the stiffer second foam element **150** located in the heel portion of the prosthetic foot **100** provides improved heel-strike cushioning, but reduces the energy return within the heel. This of course leads to an economical construction that maintains stability without sacrificing cushioning during the heel-strike.

Another advantage is in the ease and lowered costs of manufacturing the prosthetic foot **100.** For example, this design provides the ability to manufacture a prosthetic foot and cosmesis at relatively low cost using rapid molding procedures, without the need for machining structural materials or otherwise using costly casting molds and high temperature settable materials.

One method of making the prosthetic foot **100** involves assembling the pyramid **112,** pyramid adapter **114** and attachment bolts **116** together with the footplate **120** as a first assembly, then gluing the second foam element **150** to the posterior portion of the footplate **120,** molding this entire assembly within a first foam element **140,** and curing the first foam element **140** into a set and stable condition required of a foot cosmesis **130.** Of course, other molding and assembly steps could be used to achieve the same end, as would be known to one of ordinary skill in the art.

It should be noted that according to the invention, the foam matrix defining the first foam element **140** fills in the excess space within the recess **154,** for example if the preceding process is used and the recess **154** extends across the entire proximal surface of the second foam element **150,** or if the second foam element **150** is not bonded to the footplate **120** across the entire proximal surface of the second foam element **150**.

Prosthetic feet according to the present invention can be made rapidly and at relatively low cost. Such prosthetic feet have application all over the world and in particular in underdeveloped regions of the world where low cost and speed of production are critical to providing prosthetic leg and feet devices for persons of lower income where cost and simplicity is a major factor, along with appearance.

C. Detailed Description of Second and Third Embodiments

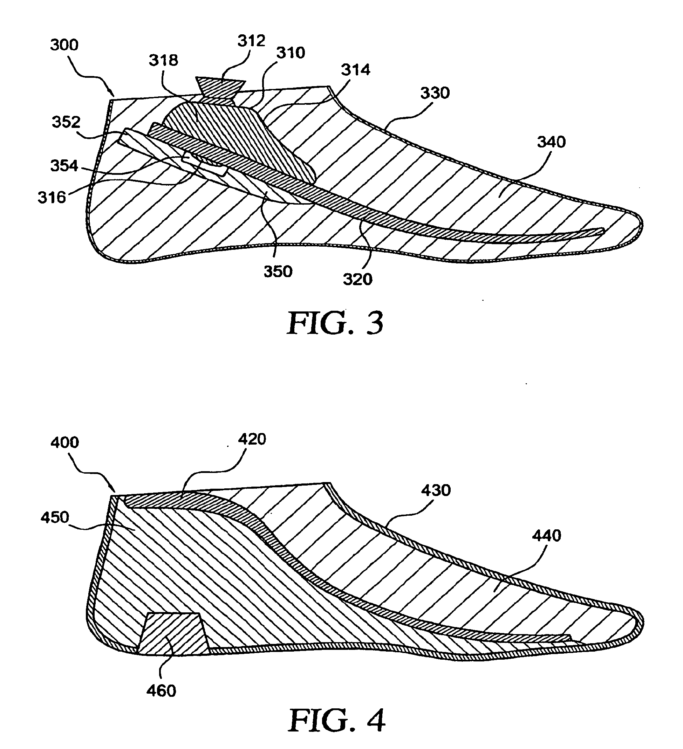

With reference to Figs. 2 and 3, alternative embodiments of a prosthetic foot **200, 300,** respectively include, a second foam element **250, 350** having a posterior extending portion **252, 352** that extends past a terminal end of the footplate **220, 320.**

The embodiments shown in Figs. 2 and 3 are similar to the embodiment shown in Fig. 1 and described above. A prosthetic foot **200, 300** has a footplate **220, 320,** of any type previously discussed, and a connection mechanism **210, 310** for connecting the prosthetic foot **200, 300** to a prosthetic limb or pylon.

The exemplary connection mechanism **210, 310** is similar to the one previously discussed above in relation to the first embodiment. The connection mechanism includes a pyramid **212, 312,** a pyramid adapter **214, 314,** and at least one attachment bolt **216, 316,** used to adjustably and firmly connect the intermediate portion **218, 318** of the connection mechanism **210, 310** to the footplate **220, 320.** Of course, any suitable attachment mechanism may be used.

The prosthetic foot **200, 300,** includes a first foam element **240, 340** and a second foam element **250, 350,** and a cosmesis shell **230, 330.** As before, the second foam element **250, 350** has a higher density, and hence a higher stiffness, than the first foam element **240, 340.** The second foam element **250, 350** also has a recess **254, 354** that performs and functions in the same manner as previously discussed.

One difference in these embodiments is that the second foam element **250, 350** has a posterior extending portion **252, 352** that extends beyond the terminal end of the footplate **220, 320.** This construction provides at least in part, the benefit of improved heel-strike cushioning and energy return.

It will be recognized that the footplate **220, 320** bears the weight of the prosthetic foot **200, 300,** and provides the toe-off characteristics desired of such feet. The second foam element **250, 350** with the posterior extending portion **252, 352** provides suitable energy storage and return for heel-strike cushion and action, including energy return as the prosthetic foot **200, 300** is rotated by normal stepping action (plantar flexion and dorsiflexion).

In the variation shown in Fig. 2, a posterior clearance space **256** is shown between the proximal surface of the second foam element **250** and the footplate **220.** This posterior clearance space **256** allows the first foam element **240** to fill in the recess and encase the bolt head **216** of the pyramid adapter **214.** This configuration allows the second foam element **250** to be bonded to the footplate **220** prior to attaching the pyramid adapter **214** to the footplate **220.**

Alternatively, as discussed above, the second foam element **250** may be more easily bonded to the footplate **220** since the bolt head **216** is accommodated in the recess, thus allowing a flush mating between the proximal surface of the second foam element **250** and the distal surface of the footplate **220.** In either case, the posterior clearance space **256** allows the first foam element **240** to fill in the recess and encase the bolt head **216** in order to remove any voids in the prosthetic foot **200.**

As can be seen in Figs. 2 and 3, the second foam element **250, 350** can have varied size and shape. A skilled artisan will recognize that the size and shape of the second foam element **250, 350** can be chosen to provide the appropriate biomechanical functions of the prosthetic foot **200, 300.**

For example, while walking on a supporting surface the prosthetic foot will cycle through contacting the supporting surface, partially contacting the supporting surface, not contacting the supporting surface, partially contacting the supporting surface, and back to contacting the supporting surface. One of the instances where the prosthetic foot is partially contacting the supporting surface is heel-strike. During heel-strike the posterior portion of the prosthetic foot will undergo compression as the user transfers their entire weight from one foot to the other. The first and second foam elements will all resist the compression force and therefore provide support for the user's weight. Due to the cellular structure of the first and second foam elements the elements will collapse in a stable manner as the compression force in the posterior portion increases.

The stable collapse of the first and second foam elements provides cushioning for the heel-strike, and energy storage and return for the remaining portion of the gait cycle. Since the second foam element has a higher density and stiffness than the first foam element it will not stably collapse at the same time as the first foam element but will stably collapse under an increased load. Thus, the energy storage and return provided by the second foam element is different from that provided by the first foam element.

As the user progresses through the gait cycle, the compression loading in the posterior portion of the prosthetic foot is reduced, while the rest of the prosthetic foot becomes subject to a compression loading until both the posterior and anterior portions of the prosthetic foot are in contact with the supporting surface, at mid-stance. The compression loading is more evenly distributed throughout the prosthetic foot at this point, but is slightly larger in the posterior portion due to the off-set location of the connection mechanism. Both the first and second foam elements have provided some energy return during the transition of the prosthetic foot from being in partial contact with the supporting surface to being in contact with the supporting surface.

During both heel-strike and mid-stance, the footplate may flex slightly, thus providing some additional energy storage and return. Still, a large amount of the energy storage and return are provided by the first and second foam elements. The footplate begins to flex to a greater degree during the next stage of partial contact, or heel-off. During heel-off, the second foam element has returned to an almost completely uncompressed state and has returned almost all of the energy that was stored therein. The anterior portion of the footplate is now in contact with the supporting surface, so the first foam element in the anterior region may be compressed to provide additional energy storage and return along with the footplate.

When the entire prosthetic foot has been lifted from the supporting surface and is no longer in contact with the supporting surface, the elements, in particular the footplate, of the prosthetic foot have provided energy return and are unstressed. Thus the cycle repeats with another heel-strike and so on.

Thus, it is evident that altering the sizes and shapes of the components of the prosthetic foot 200, 300 can affect the biomechanical properties, such as stability, energy absorption and energy return, of the prosthetic foot 200, 300. For example, the second foam element 250 in Fig. 2 has a large portion 252 extending past the terminal end of the footplate 220. This configuration provides more stability, balanced against a decrease in energy absorption and return. The embodiment of Fig. 3 provides a balance between stability and energy absorption and return by reducing the size of the second foam element **350,** and in particular the portion **352** that extends past the terminal end of the footplate **320.** The reduced size of the second foam element **350** in Fig. 3, allows a greater amount of the first foam element to be located beneath the heal portion of the footplate **320.** Thus, the embodiment of Fig. 3 provides increased energy absorption balanced against a slight decrease in stability.

It will be recognized that the discussion above detailing variations in materials and construction of the components of the prosthetic foot are equally applicable to these embodiments.

D. Detailed Description of a Fourth Embodiments

A fourth embodiment of a prosthetic foot **400** which is not part of the invention, is shown in Fig. 4. A footplate **420,** of any type previously discussed herein, is provided embedded between a first foam element **440** and a second foam element **450.** The first and second foam elements **440, 450** may be made from materials previously discussed and may have the same density and stiffness. For example, the first and second foam elements **440, 450** may be made from an ethylene vinyl acetate (EVA) closed or open cell foam having a stiffness, as measured by the hardness, in the range of 45 to 55 on the Shore A scale.

Alternatively, the second foam element **450** may have a higher density, and thus a higher stiffness than the density of the first foam element **440.** Both the first and second foam elements **440, 450** may be separately molded by known techniques and bonded to the footplate **420.** Of course, the first and second foam elements **440, 450** may also be simultaneously molded integrally around the footplate **420** in a known manner.

A cosmesis shell **430** of the type previously discussed is provided around and enclosing the first and second foam elements **440, 450,** as well as the footplate **420.** Of course, the prosthetic foot **400** may also include any of the connection mechanisms previously discussed for connecting the prosthetic foot **400** to a pylon of a prosthetic limb (not shown). The connection mechanism may be partially enclosed within the cosmesis shell **430,** as discussed above. Although not shown, the second foam element **450** can include a recess for accommodating the heads of any attachment bolts used to attach the connection mechanism to the footplate **420,** in the manner discussed above.

A third foam element **460** is provided that extends through a distal posterior surface of the cosmesis shell **430** and into the second foam element 450. The third foam element **460** as shown extends into a distal posterior portion of the second foam element **450.** The specific location and size of the third foam element **460** with respect to the second foam element **450** may be chosen in any appropriate manner in order to provide the desired biomechanical properties of the prosthetic foot **400.** For example, the width of the third foam element may be larger than the width of the footplate **420,** and nearly as wide, or as wide as the width of the cosmesis shell **430** in order to cover or nearly cover the entirety of the ground or supporting surface contact area.

The third foam element **460** can have a higher density, and hence a higher stiffness than the densities and stiffnesses of both the first and second foam elements **440, 450.** Again, all of the foam elements may be made from the same or different materials, including open or closed cell polymer foams such as closed cell polyurethane foams. Also, the size and shape of the third foam element **460** may be varied and chosen based upon the intended effect on the biomechanical properties of the prosthetic foot **400.**

This embodiment provides another low-cost prosthetic foot that performs all of the necessary functions of a prosthetic foot and is easy to manufacture using known molding techniques. As previously discussed, a stiffer heel insert provides good stability during mid-stance and terminal stance, while still providing ample cushioning and energy return during heel-strike.

F. Conclusion

These embodiments provide great flexibility for economic prosthetic feet that provide an alternative to the complex and more expensive prosthetic feet currently in use.

It is understood that the size of the prosthetic feet and the components thereof can be adjusted so that many different users having different sized feet may benefit from the present design of prosthetic feet. Specifically, the width, thickness and length of the footplates may be varied to accommodate different sized users. Accordingly, the size of the foam elements may be respectively varied along with the different sized footplates. Further, the size of the cosmesis may also be varied to surround the different sized foam elements and footplates. Exemplary considerations of cosmesis and prosthetic foot size are disclosed in U.S. patent nos. 5,800,569, 5,993,488, and 6,280,479, granted respectively on September 1, 1998, November 30, 1999, and August 28, 2001.

Of course, it is to be understood that not necessarily all objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The skilled artisan will recognize the interchangeability of various features from different embodiments. In addition to the variations described herein, other known equivalents for each feature can be mixed and matched by one of ordinary skill in this art to construct a prosthetic foot in accordance with principles of the present invention.

Although this invention has been disclosed in the context of certain exemplary embodiments and examples, it therefore will be understood by those skilled in the art that the present invention extends beyond the specifically disclosed embodiments to other alternative embodiments and/or uses of the invention and obvious modifications and equivalents thereof. Thus, it is intended that the scope of the present invention herein disclosed should not be limited by the particular disclosed embodiments described above, but should be determined only by a fair reading of the claims below.

## Claims

1. A prosthetic foot (200) comprising:
a first foam element (240) having a first stiffness and substantially defining an outer periphery of the prosthetic foot;
a resilient footplate (220) embedded within the first foam element, and having proximal and distal surfaces, and anterior and posterior portions;
a second foam element (250) embedded within the first foam element (240) and bonded to the distal surface of the posterior portion of the footplate (220), and further defining a recess (254) along a proximal surface thereof; the second foam element (250) having a second stiffness greater than the first stifthess of the first foam element (240);
wherein the recess (254) of the second foam element (250) forms an accommodation space between the proximal surface of the second foam element (250) and the distal surface of the footplate (220) wherein the accommodation space is configured to accommodate a head of an attachment bolt **characterized in that** the prosthetic foot (200) further comprises a clearance space (256) between the proximal surface of the second foam element (250) and the distal surface of the footplate (220), such that the first foam element (240) fills in the recess (254) of he second foam element (250).

2. The prosthetic foot (200) according to claim 1, further comprising:
a pyramid adapter (214);
a pyramid (212) retained by the pyramid adapter (214); and
at least a portion of the pyramid adapter (214) being embedded within the first foam element (240)

3. The prosthetic foot (200) according to claim 2, further comprising
at least one attachment bolt (216) securing the resilient foot plate (220) to the pyramid adapter (214) and the pyramid (212);
each attachment bolt (210) extending through the footplate (220) and further having a bolt head that is configured to engage the distal surface of the footplate (220); and
each bolt head accommodated within the recess (254) of the second foam element between (250) the proximal surface of the second foam element (250) and distal surface of the footplater (220)

4. The prosthetic foot (200) according to one of claims 1 to 3, wherein the second foam element (250) further includes an extending portion (252) that extends beyond a terminal end of the posterior portion of the footplate (220).

5. The prosthetic foot (200) according to one of claims 1 to 4, further comprising:
a scuff, puncture and tear resistant outer shell defining a cosmesis (230) that surrounds a substantial portion of the outer periphery.

6. The prosthetic foot (200) according, to one of claims 1 to 5 wherein the footplate (220) is a carbon or carbon fiber composite footplate.

## Patentansprüche

1. Fußprothese (200), umfassend:
ein erstes Schaumelement (240), welches eine erste Steifheit aufweist, und welches im Wesentlichen einen äußeren Umfang der Fußprothese definiert;
eine nachgiebige Fußplatte (220), welche in das erste Schaumelement eingebettet ist, und welche proximale und distale Oberflächen aufweist sowie vordere und hintere Anteile;
ein zweites Schaumelement (250), welches in das erste Schaumelement (240) eingebettet ist, und welches mit der distalen Oberfläche des hinteren Anteils der Fußplatte (220) verbunden ist, und welches weiterhin eine Aussparung (254) entlang der proximalen Oberfläche der Fußplatte definiert, wobei das zweite Schaumelement (250) eine zweite Steifheit aufweist, welche größer ist als die erste Steifheit des ersten Schaumelements (240);
wobei die Aussparung (254) des zweiten Schaumelements (250) einen Aufnahmeraum zwischen der proximalen Oberfläche des zweiten Schaumelements (250) und der distalen Oberfläche der Fußplatte (220) bildet,
wobei der Aufnahmeraum eingerichtet ist, einen Kopf eines Befestigungsbolzens aufzunehmen,
**dadurch gekennzeichnet, dass**
die Fußprothese (200) weiterhin einen Abstandraum (256) zwischen der proximalen Oberfläche des zweiten Schaumelements (250) und der distalen Oberfläche der Fußplatte (220) derart aufweist, dass das erste Schaumelement (240) die Aussparung (254) des zweiten Schaumelements (250) ausfüllt.

2. Fußprothese (200) nach Anspruch 1, weiter umfassend:
einen Pyramidenadapter (214),
eine von dem Pyramidenadapter (214) gesicherte Pyramide (212); und
wobei zumindest ein Anteil des Pyramidenadapters (214) in das erste Schaumelement (240) eingebettet ist.

3. Fußprothese (200) nach Anspruch 2, weiter umfassend:
zumindest einen Befestigungsbolzen (216), welcher die nachgiebige Fußplatte (220) an dem Pyramidenadapter (214) und der Pyramide (212) sichert;
wobei sich jeder Befestigungsbolzen (216) durch die Fußplatte (220) erstreckt und weiterhin einen Bolzenkopf umfasst, der eingerichtet ist, mit der distalen Oberfläche der Fußplatte (220) zusammenzuwirken; und
wobei jeder Bolzenkopf in der Aussparung (254) des zweiten Schaumelements (250) zwischen der proximalen Oberfläche des zweiten Schaumelements (250) und der distalen Oberfläche der Fußplatte (220) aufgenommen ist.

4. Fußprothese (200) nach einem der Ansprüche 1 bis 3, wobei das zweite Schaumelement (250) weiterhin einen Ausdehnungsanteil (252) umfasst, welcher sich über das hintere Ende des vorderen Anteils der Fußplatte (220) erstreckt.

5. Fußprothese (200) nach einem der Ansprüche 1 bis 4, weiterhin umfassend:
eine abnutzungs-, stoß- und rissresistente äußere Hülle, welche eine Kosmetik (230) definiert, die einen wesentlichen Anteil des äußeren Umfangs umgibt.

6. Fußprothese (200) nach einem der Ansprüche 1 bis 5, wobei die Fußplatte (200) eine Carbon- oder Carbonfaserverbundfußplatte ist.

## Revendications

1. Pied prothétique (200), comprenant :
un premier élément en mousse (240) présentant une première rigidité et définissant en grande partie une périphérie extérieure du pied prothétique ;
une plaque de pied résiliente (220) incorporée à l'intérieur du premier élément en mousse, et présentant des surfaces proximale et distale, et des parties antérieure et postérieure ;
un second élément en mousse (250) incorporé à l'intérieur du premier élément en mousse (240) et lié à la surface distale de la partie postérieure de la plaque de pied (220), et définissant en outre un retrait (254) le long d'une surface proximale de celle-ci, le second élément en mousse (250) présentant une seconde rigidité supérieure à la première rigidité du premier élément en mousse (240) ;
dans lequel le retrait (254) du second élément en mousse (250) forme un espace de réception entre la surface proximale du second élément en mousse (250) et la surface distale de la plaque de pied (220), dans lequel l'espace de réception est configuré de manière à recevoir une tête d'un boulon de fixation, **caractérisé en ce que** le pied prothétique (200) comprend en outre un espace de jeu (256) entre la surface proximale du second élément en mousse (250) et la surface distale de la plaque de pied (220), de sorte que le premier élément en mousse (240) remplit le retrait (254) du second élément en mousse (250).

2. Pied prothétique (200) selon la revendication 1, comprenant en outre :
un adaptateur pyramide (214) ;
une pyramide (212) retenue par l'adaptateur pyramide (214), et
au moins une partie de l'adaptateur pyramide (214) étant incorporée à l'intérieur du premier élément en mousse (240).

3. Pied prothétique (200) selon la revendication 2, comprenant en outre :
au moins un boulon de fixation (216) fixant la plaque de pied résiliente (220) sur l'adaptateur pyramide (214) et la pyramide (212) ;
un boulon de fixation (216) s'étendant à travers la plaque de pied (220) et présentant en outre une tête de boulon configurée pour venir en prise avec la surface distale de la plaque de pied (220), et
chaque tête de boulon reçue à l'intérieur du retrait (254) du second élément en mousse (250) entre la surface proximale du second élément en mousse (250) et la surface distale de la plaque de pied (220).

4. Pied prothétique (200):selon l'une quelconque des revendications 1 à 3, dans lequel le second élément en mousse (250) inclut en outre une partie d'extension (252) qui s'étend au-delà d'une extrémité terminale de la partie postérieure de la plaque de pied (220).

5. Pied prothétique (200) selon l'une quelconque des revendications 1 à 4, comprenant en outre :
une coque extérieure résistant aux frictions légères, aux perforations et déchirures définissant un recouvrement esthétique (230) qui entoure une partie significative de la périphérie extérieure.

6. Pied prothétique (200) selon l'une quelconque des revendications 1 à 5, dans lequel la plaque de pied (220) est une plaque de pied composite en carbone ou fibre de carbone.
